Europäisches Patentamt

European Patent Office

Office européen des brevets

(11) Publication number: **0 293 792**
**A2**

## (12) EUROPEAN PATENT APPLICATION

(21) Application number: 88108569.0

(22) Date of filing: 28.05.88

(51) Int. Cl.⁴: **C12N 15/00 , C12N 1/20 , A61K 39/21 , G01N 33/569**

The applicant has filed a statement in accordance with Rule 28 (4) EPC (issue of a sample only to an expert). Accession number(s) of the deposit(s): ATCC 67378.

(30) Priority: 01.06.87 US 57569

(43) Date of publication of application:
07.12.88 Bulletin 88/49

(84) Designated Contracting States:
AT BE CH DE ES FR GB GR IT LI LU NL SE

(71) Applicant: **E.I. DU PONT DE NEMOURS AND COMPANY**
**1007 Market Street**
**Wilmington Delaware 19898(US)**

(72) Inventor: **Ferguson, Blair Quinn**
**2513 W. 18th Street**
**Wilmington Delaware 19806(US)**
Inventor: **Tribe, David Edward**
**431 Dean Drive**
**Kennett Square Pennsylvania 19348(US)**
Inventor: **Petteway, Stephen Robert**
**1017 Ashley Street**
**West Chester Pennsylvania 1382(US)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) Human immunodeficiency virus GAG-encoded proteins.

(57) The invention concerns nonfused recombinant proteins corresponding to the complete HIV gag and the complete HIV p17$^{gag}$ subregion coding sequences, which are useful immunological components of diagnostics, therapeutics, and vaccines.

FIG. 2

EP 0 293 792 A2

# Human Immunodeficiency Virus gag-encoded Proteins

## FIELD OF THE INVENTION

The present invention concerns proteins derived by molecular cloning that are useful immunological components of diagnostics, therapeutics, and vaccines for infectious diseases. In particular, the invention relates to proteins encoded by the gag gene of human immunodeficiency virus, the etiologic agent of acquired immunodeficiency syndrome.

## BACKGROUND

The human immunodeficiency virus (HIV, also LAV, HTLV-III, or ARV) is the primary etiologic agent of the acquired immune deficiency syndrome (AIDS) [Barre-Sinoussi et al., Science 220:868-871 (1983); Gallo et al., Science 224:500-503 (1984); Levy et al., Science 225:840-842 (1984)]. The underlying disease state involves a tropism of HIV for the T4+ lymphocyte subset resulting in a selective depletion of the helper/inducer cells of the immune system, leaving the HIV-infected individual defenseless against a number of opportunistic infections. Approximately two million people in the United States and five million or more individuals world-wide may now be infected by HIV. The U.S. Public Health Service now estimates that the cost of caring for AIDS patients in 1991 will be between$8 billion to $16 billion per year. Thus, the development of diagnostics, therapeutics, and vaccines to HIV is the subject of intense medical research.

The nucleotide sequence of several independent viral isolates of HIV have been determined [Ratner et al., Nature 313:277-284 (1985); Muesing et al., Nature 313:250-258 (1985); Wain-Hobson et al., Cell 40:9-17 (1985); Sanchez-Pescador et al., Science 227:484-492 (1985)]. HIV is related to the lentivirus subgroup of retroviruses. Lentiviruses are associated with slowly developing, non-tumorigenic diseases in other animal systems. The major structural proteins of the HIV infectious particle are encoded by the viral gag, pol, and env genes. In addition to the gag,pol, and env genes, the genome of HIV contains several other open reading frames designated sor,tat, art/trs and 3'-orf which encode additional viral proteins. These proteins are known to serve important regulatory functions during the HIV infectious cycle.

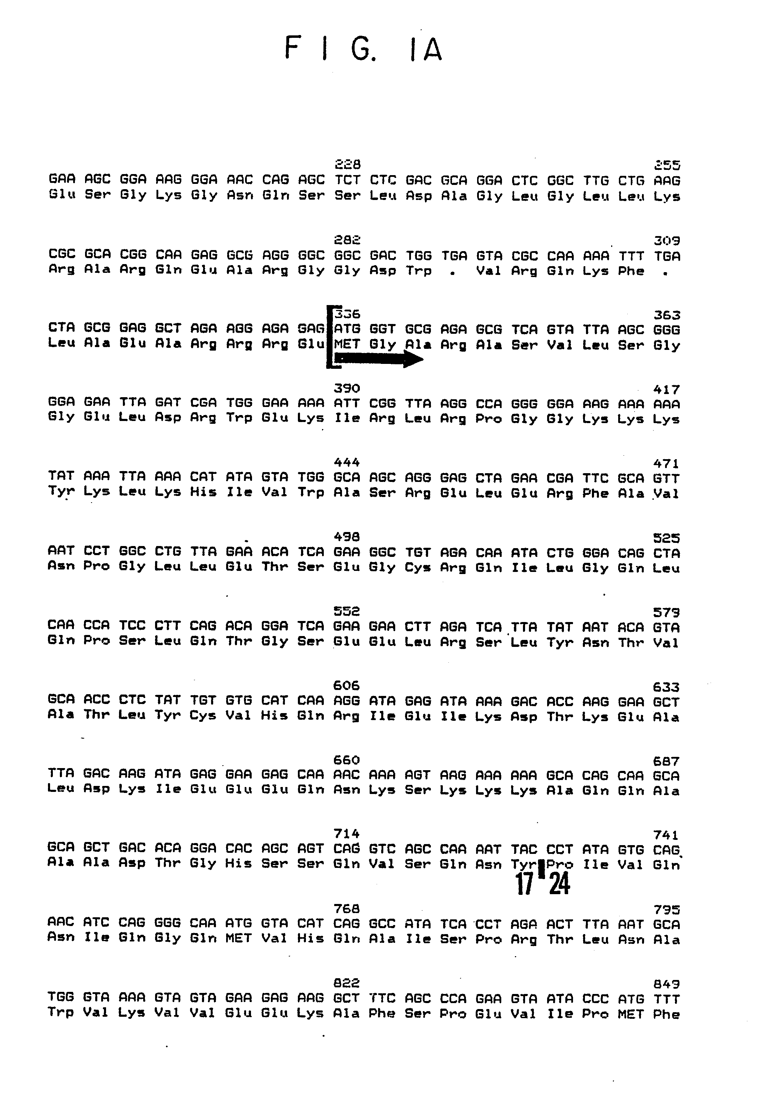

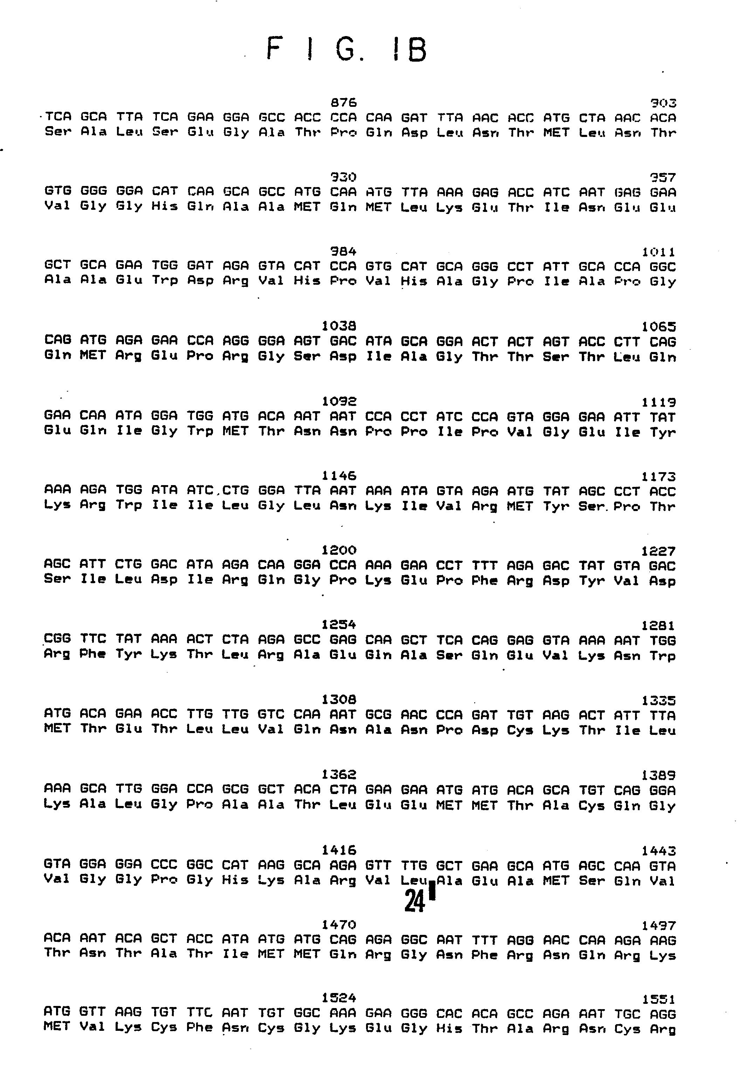

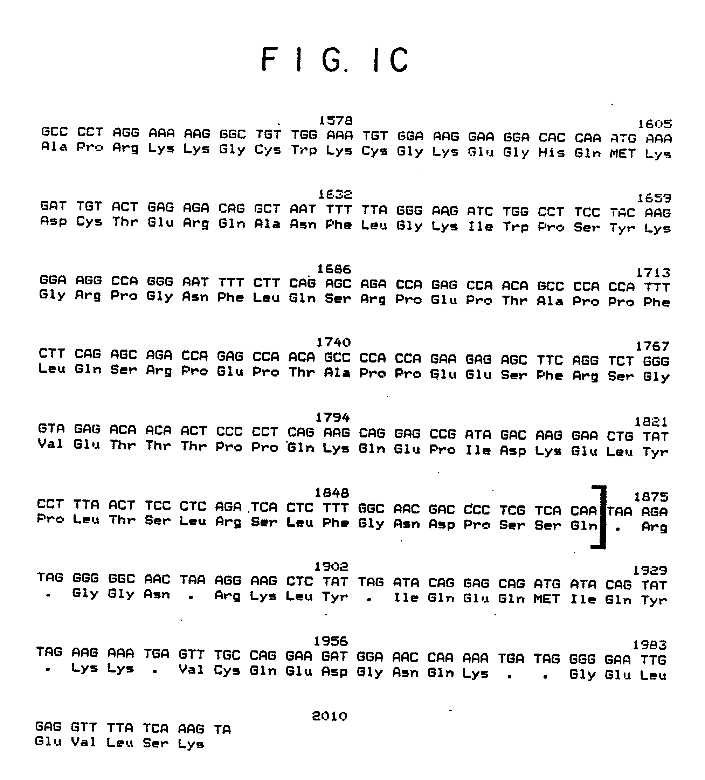

The gag gene products are involved in the structure of the genomic RNA-containing core and the assembly of the virus. The gag gene is termed "gag" for group-specific antigen, since in other retroviral systems the analogous gene encodes proteins that are cross- reactive with similar proteins of a related group of retroviruses. The HIV gag gene encodes a precursor of about 55,000 daltons (55 kDa) which is designated p55$^{gag}$. The gag coding sequence of HIV isolate BH10 (HIV$_{BH10}$) (Ratner et al., op. cit.) is contained within nucleotides 334 to 1869 and is comprised of 512 codons (Figure 1). The nucleotide numbering is according to Ratner et al. (op. cit.).

During the HIV infectious life cycle, the gag precursor p55$^{gag}$ is proteolytically processed into species of approximately 24, 17, and 15 kDa. The gag proteins are designated p24$^{gag}$, p17$^{gag}$, and p15$^{gag}$. The mature gag proteins are derived from subregions of the p55$^{gag}$ precursor in the order N-p17-p24-p15-C (N- and -C designate the amino and carboxyl terminus of the protein, respectively).

As is the case for the N-terminal gag-encoded proteins of other mammalian retroviruses [Henderson et al., Proc. Natl. Acad. Sci. 80:339-343 (1983); Rein et al. Proc. Natl. Acad. Sci. 83:7246-7250 (1986)], the N-terminal gag-encoded p17$^{gag}$ is modified at its mature N-terminal glycine by myristylation. The N-terminal lipid myristic acid moiety of p17$^{gag}$ presumably mediates the interaction of p17$^{gag}$ with the lipid membrane of the virus particle. Gag-derived determinants are known to be exposed on the surface of cells infected by other mammalian retroviruses [Schiff-Maker and Rosenberg, Virology 154:286-301 (1986)]. Thus, HIV p17$^{gag}$-specific epitopes may be present on the surface of infectious HIV particles and HIV-infected cells. The p24$^{gag}$ protein is the major virus capsid or core protein. The C-terminal gag-encoded p15$^{gag}$ protein is highly basic and likely constitutes the core ribonucleoprotein and binds nonspecifically to many sites on the viral RNA.

Earlier reports describe the expression of subregions of HIV gag protein in heterologous expresion systems such as in E. coli [Dowbenko et al., Proc. Natl. Acad. Sci. 82: 7748-7752 (1985); Chang et al., Science 228:93-99 (1985); Ghrayeb et al., DNA 5:93-99 (1986); Steimer et al., Virology 150: 283-290 (1986); Shoeman et al., Anal. Biochem. 161:370-379 (1987)] and in yeast [Kramer et al., Science 231:1580-1584 (1986)]. These recombinant gag-derived proteins produced in heterologous expression systems were shown to be useful as antigens for the detection of HIV gag-specific antibodies in human sera. No previous report has described the production using a heterologous expression system, for example an E. coli plasmid expression system, of proteins corresponding to the full length, nonfused gag precursor p55$^{gag}$ or to the mature gag subregion p17$^{gag}$. The present invention provides the full length nonfused HIV gag precursor p55$^{gag}$ and the mature gag subregion p17$^{gag}$ as produced using a heterologous expression system.

## SUMMARY OF THE INVENTION

The invention provides proteins corresponding to the full length, nonfused HIV gag precursor p55$^{gag}$ and the mature nonfused p17$^{gag}$ subregion of p55$^{gag}$ which are produced using molecular cloning methods and heterologous expression vector systems. Polypeptides corresponding to p55$^{gag}$ and p17$^{gag}$ of the HIV isolate BH10 (Ratner et al., op. cit.) were expressed in E. coli. Protein p55$^{gag}$ is encoded by nucleotides number 334 to 1869 (nucleotide numbering according to Ratner et al., op. cit.) (see Figure 1). The p17$^{gag}$ segment of p55$^{gag}$ is encoded by nucleotides number 334 to 729. The HIV gag proteins are useful immunological components of diagnostics to identify individuals and blood products that have been exposed to HIV, of reagents that can be used to monitor and stage the progress of the disease in clinical therapeutic trials of therapeutic agents able to control the disease, and of a vaccine that will protect iduals who are exposed to HIV.

## DETAILED DESCRIPTION OF THE INVENTION

Two recombinant peptides containing only amino acid sequences corresponding to the gag coding sequence of HIV have been created. These recombinant gag-derived polypeptides are highly immunoreactive with the many sera from individuals infected with HIV. The term "peptide" is well known in the art and refers to a compound of two or more amino acids. One of the peptides of the invention corresponds to the complete and nonfused HIV gag p55$^{gag}$ precursor protein. In HIV$_{BH10}$, p55$^{gag}$ is encoded by nucleotides 334 to 1869 (nucleotide numbers are according to Ratner et al., op. cit.). A second peptide of the invention corresponds to the mature, N-terminal gag-encoded protein, p17$^{gag}$. In HIV$_{BH10}$, p17$^{gag}$ is encoded by nucleotides 334 to 729 (Ratner et al., op. cit.). The specific domains of HIV are known in the art.

The present E. coli-expressed proteins corresponding to HIV p55$^{gag}$ and p17$^{gag}$ are referred to as GAG55 and GAG17, respectively. The structures of plasmid expression vectors designed for the expression of GAG55 and GAG17 in E. coli are detailed in Example 1.

As used herein, the term "expression vector" includes a DNA molecule which contains signals, recognized by a particular host biological cell, that direct and determine the expression of a particular gene sequence in the host cell. The vector DNA may, for example, contain information that determines the uptake of the vector DNA by the host cell, the integration and replication of the vector DNA, and the corresponding RNA biosynthesis and translation. Examples of expression vectors include recombinant viruses, plasmids and genes.

It is to be understood that the term "corresponding to" includes modifications of the specified amino acid sequences which do not adversely affect the antigenic characteristics of the peptide of the invention. For example, different isolates of HIV are known to differ to some extent in the predicted amino acid sequence of the gag-derived proteins (Ratner et al., op. cit.; Muesing et al., op. cit.; Wain-Hobson et al., op. cit.; Sanchez-Pescador, op. cit.). One skilled in the art could align the amino acid sequences of peptides from different sources to the schematic of Figure 1 or the nucleotide sequence of Ratner et al. (op. cit.) to identify the segments therein which correspond to the peptides defined herein.

HIV gag-derived proteins are useful immunological components of diagnostics, therapeutics, and vaccines for HIV-related disease, including acquired immune deficiency syndrome (AIDS). HIV-expressed gag proteins are known to be useful immunological antigens for the detection of HIV gag-specific antibodies [Petricciani, Ann. Int. Med. 103:726-729 (1985)]. The analysis of HIV-specific antibodies in human serum samples is extremely important in diagnosis of disease and for screening of human blood products. An enzyme-linked immunosorbent assay (ELISA) which uses HIV grown in tissue culture cells as antigen is now widely used for detecting sera positively immunoreactive to HIV (Petricciani, op. cit.). The ELISA derived from purified HIV particles grown in tissue culture is designated HIV-ELISA.

Since HIV infection leads to a series of clinical manifestations accompanied by variable antibody levels to different antigens, useful blood screening and diagnostic reagents should contain multiple antigens. Moreover, it is desirable to detect and quantitate antibody levels to specific antigenic components of HIV. Although the HIV-ELISA is a sensitive tool for the detection of HIV-specific antibodies in human serum samples, this test does not provide information as to which antigenic components of HIV are reactive with human antibodies.

The production of individual defined HIV-derived proteins, such as gag-encoded proteins, in E. coli provides antigen reagents for the selective detection and quantitation of antibodies recognizing specific antigens, such as gag-encoded antigens. HIV gag proteins derived by molecular cloning and expressed in heterologous expression systems, i.e., recombinant gag proteins, are useful for the development of novel HIV diagnostic kits. For example, the E. coli produced HIV GAG55 and GAG17 proteins can be efficiently utilized in a diagnostic ELISA for the detection and quantitation of HIV gag-specific antibodies that are associated with prior infection with HIV.

A heterologous expression system such as that utilizing an E. coli plasmid expression vector, provides a preferred alternative to infectious HIV grown in tissue culture as a source of antigen for ELISA blood screening and diagnostic reagents. Importantly, the E. coli plasmid expression system is free of infectious HIV. Moreover, HIV gag-derived proteins GAG55 and GAG17 can be expressed at high levels in E. coli using the appropriate vectors, thereby facilitating the purification of these useful polypeptides. Thus, recombinant gag can be produced in large quantities and avoids the biohazard and containment problems associated with the production of gag proteins from infectious HIV virions.

The recombinant gag proteins of the present invention are preferred to previously described recombinant gag proteins. The recombinant product GAG55 corresponds to the full length, non-fused product of the complete gag coding sequence. Previously described recombinant gag proteins only contain subregions of gag. For example, in a recombinant gag protein described by Kramer et al. (op. cit.) and Shoeman et al. (op. cit.), amino acids number 2 to 12 of p55$^{gag}$ are deleted. Other reported recombinant gag proteins reported have larger segments of the p55$^{gag}$ sequence deleted (Dowbenko et al., op. cit.; Chang et al., op. cit.; Ghrayeb et al., op. cit.; Steimer et al., op.cit.). More gag-specific antigenic determinants are represented by the product of the complete, full length gag coding sequence than are represented by incomplete subregions of gag. Thus, the product of the full length gag coding sequence, such as GAG55, is expected to be a more sensitive reagent for the detection of gag-specific antibodies than are antigens comprised of only subregions of gag.

In particular, GAG55 and GAG17 contain the region of gag corresponding to the complete p17$^{gag}$. This complete region of gag is not present in other reported recombinant gag proteins. As discussed previously and below, the p17$^{gag}$ region of gag contains antigenic determinants that may be presented on the surface of HIV or HIV-infected cells, and may be critical for a protective host immune response to HIV.

Another advantage of the peptides of the invention is that the recombinant gag-derived proteins are not fused to other, non-HIV, or non-HIV gag sequences. The inclusion of additional non-HIV sequences, such as E. coli-derived sequences, fused to the HIV gag coding sequence is not desirable since these sequences could lead to frequent false-positive (i.e., non-HIV) antibody reactivity. Thus, the HIV gag sequences have been expressed in E. coli in the absence of fused bacterial or other non-HIV protein sequences. It is also desirable to express nonfused authentic gag proteins, rather than fusion proteins, in order to facilitate the correct folding and native structure of the product. The correctly folded structure of the product may be critical to its effectiveness as a diagnostic, therapeutic, or vaccine reagent. Previously reported recombinant HIV gag-derived proteins are fusion polypeptides containing non-HIV sequences.

Although most current strategies for the development of an AIDS vaccine are focusing on the HIV env gene, the gag antigens may well be an important component of a protective vaccine against HIV. Indeed, recent reports suggest that gag p17$^{gag}$ antigenic determinants are accessible to the neutralizing effects of specific antibodies [Sarin et al., Science 232: 1135- 1137 (1986)]. As discussed previously, p17$^{gag}$ determinants may be exposed on the envelope of the virus itself, since the gag p17$^{gag}$ protein is thought to be associated with the virus lipid membrane.

AIDS patients are less likely to have antibodies to gag-encoded proteins compared to individuals with milder forms of the disease [Steimer et al., Virology 150: 283-290 (1986)]. Thus, there is at least correlative evidence that the gag-specific host immune response may be critical for the effective control of HIV by an HIV-exposed individual.

Due to the heterogeneity in the sequence of env from various HIV isolates [Ratner et al., Nature 313:277-284 (1985); Wain-Hobson et al., Cell 40:9-17 (1985); Muesing et al., Nature 313:250-258 (1985); Sanchez-Pescador et al., Science 227:484-492 (1985)], it is possible that a vaccine containing env from a single HIV isolate may not have a broadly neutralizing effect on other HIV species. The degree of heterogeneity in the gag gene product among different HIV isolates is far less than that found for env [Ratner et al., Nature 313:277-284 (1985); Muesing et al., Nature 313:250-258 (1985); Wain-Hobson et al., Cell 40:9-17 (1985); Sanchez-Pescador et al., Science 227:484-492 (1985)]. Thus, HIV gag gene products may be an important component of a vaccine that is broadly protective against many isolates of HIV.

Since the peptides of the invention are produced by recombinant DNA technology in the absence of infectious HIV, they provide several advantages over more traditional vaccine approaches using killed or attenuated viral preparations. Recombinant peptides are safe to prepare and administer and no genetic information is introduced by vaccination. In addition recombinant peptides are more readily produced and purified than the viral-expressed peptides isolated from virus-infected cells.

In one embodiment, the peptide of the invention is used in a diagnostic kit used for detecting antibodies to HIV in a biological sample. The peptide can be employed in a process for detecting antibodies to HIV comprising contacting a biological sample with the peptide and detecting immunoreactivity. The peptide can also be employed as a component in a vaccine protective against HIV. The vaccine comprises an effectively protective amount of the peptide.

E. coli strain KA1298 transformed with plasmid pGAG55, encoding protein GAG55 and described in Example 1, has been deposited with the American Type Culture Collection (ATCC), Rockville, Maryland, and bears deposit accession number 67378. This deposit is available to the public upon the grant of a patent to the assignee. However, it should be understood that the availability of a deposit does not constitute a license to practice the subject matter in derogation of patent rights granted by governmental action.

Materials and Methods

Unless otherwise specified, parts and percentages are by weight and degrees are Celsius.

Plasmid Construction

Plasmid constructions were carried out using standard methodology as described by Maniatis et al., Molecular Cloning: A Laboratory Plasmid, Cold Spring Harbor Laboratory, New York (1982), the teaching of which is hereby incorporated by reference. Enzymes and other reagents used for plasmid constructions were obtained from Bethesda Research Laboratories, Gaithersburg, MD or New England Biolabs, Beverly, MA. Methods for digesting, identifying, recovering, and purifying the various nucleotide sequences used in the invention are known to those skilled in the art as are methods for ligating the sequences into vectors, transforming host microorganism strains, cloning, and recovering products synthesized. Accordingly, the methods will only be described by reference to specific embodiments of the invention set forth hereinafter.

E. coli Strains, Plasmid Expression Vectors, and Induction of Expression

Several types of E. coli plasmid expression vectors were used for the production of HIV gag-derived proteins in E. coli. In the vectors used, expression of the desired product is controlled transcriptionally using either the operator and promoter of the E. coli tryptophan operon [Ivanoff et al., Proc. Natl. Acad. Sci. 83:5392-5396 (1986)] or the phage lambda $P_L$ promoter [Rosenberg et al., Methods Enzymol. 101:123-138 (1983)]. We have also used the E. coli lactose operon promoter to direct expression of HIV gag-derived proteins in E. coli.

Plasmid vectors utilizing the phage lambda promoter were derived from plasmid pBF106. The E. coli plasmid expression vector pBF106 was constructed as follows. The small HpaI to BamHI fragment of plasmid pKC30 [Rosenberg et al., Methods Enzymol. 101:123-138 (1983); Rao, Gene 31:247-150 (1984)] (obtained from K. Abremski, Du Pont Experimental Station, Wilmington, DE) was replaced with the following oligonucleotide linker:

5´- AAC GAA TCC GAA GTG TAA GCC ATG -3´
3´- TTG CTT AGG CTT CAC ATT CGG TAC CTAG -5´

The inserted sequence provides signals for efficient translation initiation and a unique NcoI restriction endonucleose site. Since the translation start ATG forms part of the NcoI site, coding sequences can easily be fused in-frame directly to the ATG and translation initiation signal.

Expression from the $P_L$-containing plasmids is controlled transcriptionally using a lysogenic host that provides lambda cI repressor. Expression is induced by inactivating cI using nalidixic acid [Mott et al., Proc. Natl. Acad. Sci. 82:88-92 (1985)] or by temperature-shift and using a temperature sensitive repressor such as cI857 [Rosenberg et al., Methods Enzymol. 101:123-138 (1983)]. Temperature-shift inductions were carried out using host strain KA1298 (obtained from K. Abremski, Du Pont Experimental Station, Wilmington, DE), according to the procedure of Young et al., Proc. Natl. Acad. Sci., 80:6105-6109 (1983). KA1298 is a defective lambda lysogenic containing cI857. Nalidixic acid inductions were carried out using the defective lysogenic host DC550 (obtained from D. Court, NCI, Frederick, MD), according to Mott et al., Proc. Natl. Acad. Sci. 82:88-92 (1985).

E. coli plasmid expression vectors containing the tryptophan promoter were derived from pKGP36.trp (Ivanoff et al., op. cit). E. coli host strains MM294 and HB101, DH5 (F⁻ recA1, endA1, gyrA96, hsdR17, supE44), DH5Δ (lac), SC122, CAG456(htpR165), CAG629(lon-htpR165), and SG4119 (Δlon) were used [Maniatis et al., Molecular Cloning: A Laboratory Manual, Cold Spring Harbor Laboratory, NY (1982); Baker et al., Proc. Natl. Acad. Sci. 81:6779-6783]. Expression from the trypophan promoter was carried out as described (Ivanoff et al., op. cit.).

## Serum Samples and Serology

Human sera were collected at Blood Bank of Delaware blood donor centers during 1986 or were obtained from T. Mathews, Duke University. Serum was stored at -70°C before testing, and repeated freezing and thawing was avoided. A preliminary classification of all sera was made based on repeated tests with an HIV-ELISA (Du Pont, Wilmington, DE) blood screening kit, on the results of HIV immunoblot testing carried out by a commercial laboratory (Biotech Research Laboratories, Rockville, MD), and on testing sera for reactivity to env using a recombinant antigen (ENV9) based ELISA (described below). Procedure 1, the 3-1/2 hour assay described in the Du Pont HTLV- III ELISA blood screening kit, was used to test sera. Further immunoblot analysis was carried out using commercially available HIV antigen-containing nitrocellulose strips (Du Pont-Biotech Research Laboratories).

## Immunoblot Analysis

Standard methods for polyacrylamide gel electrophoresis (SDS-PAGE) [Laemmli, Nature 227:680-685 (1970)] and electrophoretic transfer of proteins to nitrocellulose [Towbin et al., Proc. Natl. Acad. Sci. 27:4350-4354 (1979)] were used. Crude preparations of GAG55 (30 ng of total E. coli protein per lane were typically used for immunoblot analysis. Following electrophoretic transfer, the nitrocellulose strip were preincubated 30 minutes at room temperature in Blotto buffer (5% dried non-fat milk solids, 0.05% Tween 20, 0.04% normal goat serum, phosphate buffered saline). The strips were then incubated with a dilution (generally 1:50 in Blotto buffer) of sample serum or antibody for 1 hour to overnight at 4°C. The nitrocellulose strips were then washed exhaustively with phosphate buffered saline containing 0.05% Tween-20. Human immunoglobulins were detected usually using biotinylated anti-human immunoglobulin, and avidinD-horseradish peroxidase reagents (Vector Laboratories, Burlingame, CA). Preabsorption of sera for competition experiments involved incubation of 10 to 20 μl of serum with 1 to 3 volumes of E. coli extract (containing 5 mg/ml of bacterial protein) for 2 hours at 25°C followed by centrifugation to remove insoluble material.

## Protein Purification

E. coli-expressed GAG55 was purified by immunoaffinity chromatography. Induced E. coli were collected by centrifugation and the cell pellet was stored at -70°C. The induced E. coli cell pellet was suspended in PBS and the cells were lysed using a French press. The lysate was centrifuged (27000 x g) and the supernatant applied to an immunoaffinity column containing p17$^{gag}$ mouse monoclonal-specific antibody BT2 (Biotech Research Laboratory, Rockville, MD) covalently attached to Reacti-gel (Pierce Chemical, Rockford, IL). The immunoaffinity column was prepared as specified by the supplier and washed and equilibrated with phosphate buffered saline (PBS). The E. coli cell lysate was loaded onto the column and the column was extensively washed with PBS. Fractions containing gag protein were eluted from the column using 0.5 N acetic acid and were then pH neutralized, lyophilized, and dissolved in PBS containing 0.1% SDS.

Alternatively, GAG55 was purified from a French press lysate of E. coli by cation exchange chromatography. Soluble proteins were dialyzed against 0.05 M phosphate buffer (pH 7.0), applied to a Pharmacia Mono S column (Pharmacia, Piscataway, NJ) and eluted with a NaCl gradient. The purity of the antigen was approximately 90% purified.

The peptide ENV9 was purified from E. coli containing the vector pENV9, as discussed in assignee's copending application, serial number 010056, filed Feb. 2, 1987. The predicted protein product of pENV9, designated ENV9, contains 340 amino acids which correspond to 54 amino acids from the N-terminal of the poliovirus sequence of pEXC [Ivanoff et. al., op. cit.], 46 amino acids of the C-terminal of the HIV env gp120 domain and 340 amino acids of the N-terminal of the env gp41 domain. Enzyme Linked Immunosorbant Assay (ELISA)

Purified GAG55 antigen in 60 mM Na$_2$CO$_3$ pH 9.6 buffer containing 0.0005% SDS was applied to Immulon II microtiter plates at 100 ng/well at 4°C for about 18 hours. Purified ENV9 antigen was similarly applied in 60 mM carbonate pH 9.6 buffer containing 0.00006% SDS at a concentration of 20 ng/well. ELISA procedures were carried out as specified for the commercial HIV-ELISA kit (Du Pont, Wilmington, DE). The antigen-coated plates were washed with PBS + 0.05% Tween 20 (PBS-T). Plate washings were performed using 3 cycles on a Titertek Microplate Washer 120 followed by rotating the plate and washing again. The plates were incubated with PBS-T for 1 hour at 37° and were then washed 3 times with PBS-T and stored dry at 4° until they were used.

The plates were incubated with patient sera at a 1:20 to 1:100 dilution in diluent (PBS-T + 5% bovine serum albumin, 20% heat inactivated normal goat serum (NGS), 0.1% sodium azide, 0.05% thimerosal) in the microtiter wells and incubated for 2 hours at 25°. The plates were then washed with PBS-T, exposed to goat anti-human IgG conjugated to alkaline phosphatase (Jackson ImmunoResearch, West Grove, PA) for 1 hour at 25° and washed with PBS-T. The alkaline phosphatase reaction was developed, as specified by the supplier, by exposure to 72 μg para-nitrophenylphosphate in 100 μl of diethanolamine buffer (2 M, pH9.8) with magnesium chloride and 0.02% sodium azide at a for 30 minutes at 37° followed by addition of sodium hydroxide at a concentration of 1 N. The plates were read on a Titertek Multiskan MCC microtiter plate reader at a wavelength of 405 nm.

BRIEF DESCRIPTION OF FIGURES

FIGURE 1: Nucleotide sequence of the gag region of HIV$_{BH10}$ (Ratner et al., op. cit.). The start of the gag p55$^{gag}$ coding sequence is indicated by an open bracket and arrow; the end of the gag p55$^{gag}$ coding sequence is indicated by a closed bracket. The junction between the p17$^{gag}$ and p24$^{gag}$ polypeptides, that arise from proteolytic processing of p55$^{gag}$, is indicated. Protein p55$^{gag}$ is comprised by gag amino acids number 1 to 512; p17$^{gag}$ is comprised by gag amino acids number 1 to 132.

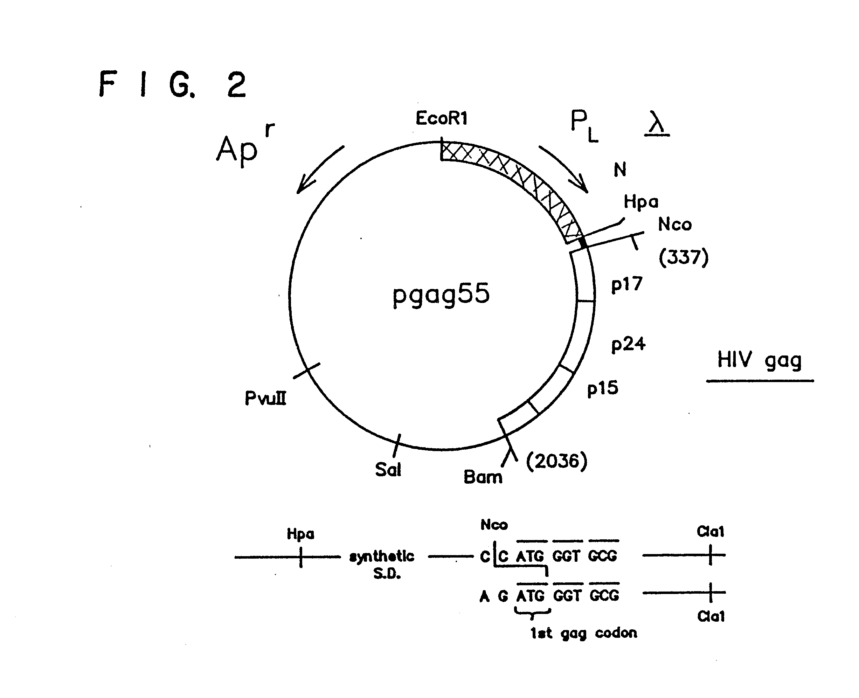

FIGURE 2: Schematic representation of E. coli plasmid vector designed to express full length, non-fused HIV gag precursor protein p55$^{gag}$. Plasmid pGAG55 (pgag55) contains sequences derived from pBR322 (thin line), phage lambda (thick cross-shatched line), and HIV$_{BH10}$ (thick open line), as well as a chemically synthesized DNA segment (thick closed line). In pGAG55, expression of the gag coding sequence is controlled by the phage lambda P$_L$ promoter and translation initiation signals derived from a chemically synthesized DNA sequence. As indicated, the two nucleotides 5' and preceding the gag ATG initiation codon in HIV$_{BH10}$ have been changed from AG to CC, thereby generating a unique NcoI restriction endonuclease site.

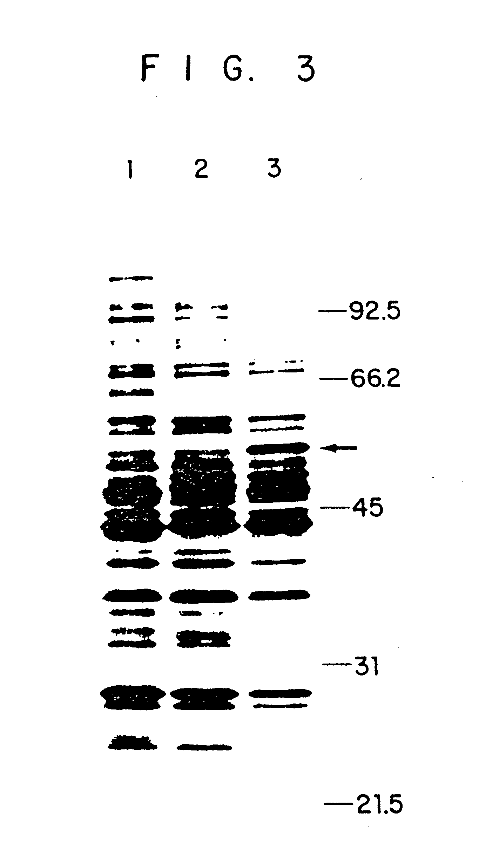

FIGURE 3: Expression of GAG55 in E. coli. The accumulation of GAG55 in E. coli was analyzed by SDS-PAGE of total E. coli lysates and Coomassie staining. Lane 1: E. coli DC550 containing pBF106 (DC550/pBF106) induced with nalidixic acid. Lane 2: DC550/pGAG55, non-induced. Lane 3: DC550/pGAG55, induced with nalidixic acid. The GAG55 protein represents approximately 5% of total cell protein in the nalidixic acid-induced DC550 cells containing pGAG55 (lane 3).

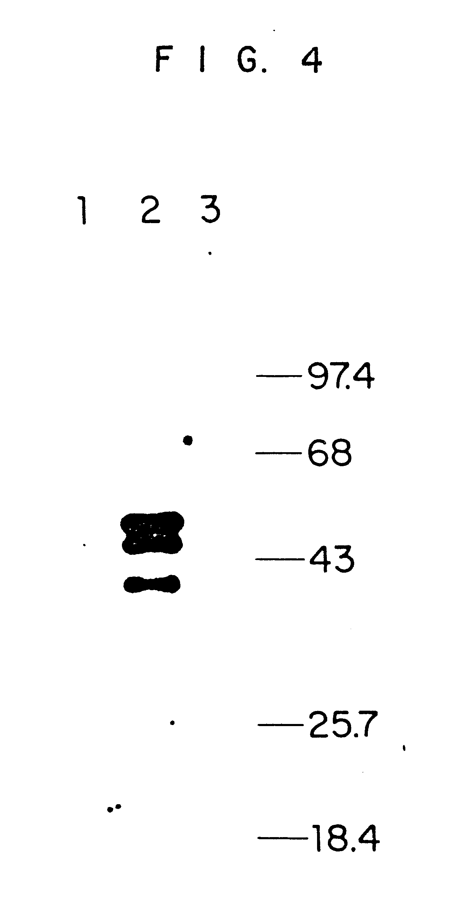

FIGURE 4: Immunoblot reactivity of E. coli-expressed GAG55-derived proteins with human AIDS patient serum antibodies. Total E. coli lysates were analyzed. Lane 1: DC550/pGAG55, non-induced; lane 2: DC550/pGAG55 nalidixic acid-induced; lane 3: DC550/pBF106, nalidixic acid-induced.

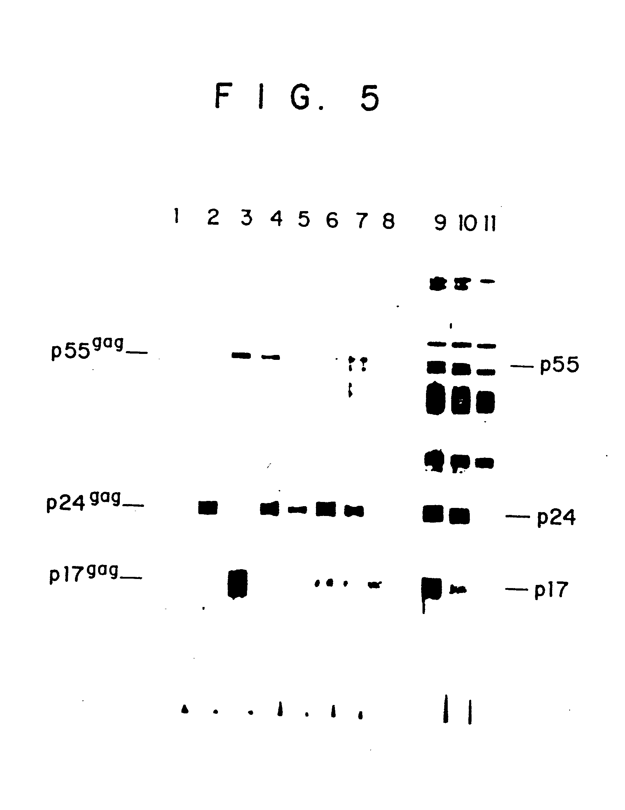

FIGURE 5: Binding of gag-specific antibodies in human HIV-seropositive sera by E. coli-expressed GAG55, as analyzed by competition binding and immunoblot. Prior to immunoblot of HIV antigens (Biotech Research Labs, Rockville, MD), human HIV-seropositive sera were preincubated with cell extracts prepared from induced E. coli, as indicated. Lane 9: DC550/pBF106, 20 μl extract; lane 10: DC550/pGAG55, 5 μl extract; lane 11: DC550/pGAG55, 20 μl extract. As shown, E. coli-expressed GAG55 protein effectively and selectively binds to all of the immunoblot-detectable gag-specific antibodies in this AIDS patient serum. Similar results have been obtained for several other HIV-seropositive sera. Thus, essentially all of the HIV gag-derived determinants and epitopes recognized by human antibodies are presented by E. coli-expressed GAG55. The figure also illustrates the identification of human serum samples containing HIV gag-reactive, HIV env- and pol-nonreactive antibodies. Human sera were analyzed by immunoblot of HIV antigens. In lanes 2 to 8, the specificity of the immunoblot reactivity for gag antigen was confirmed by competition with E. coli-expressed GAG55 protein (not shown).

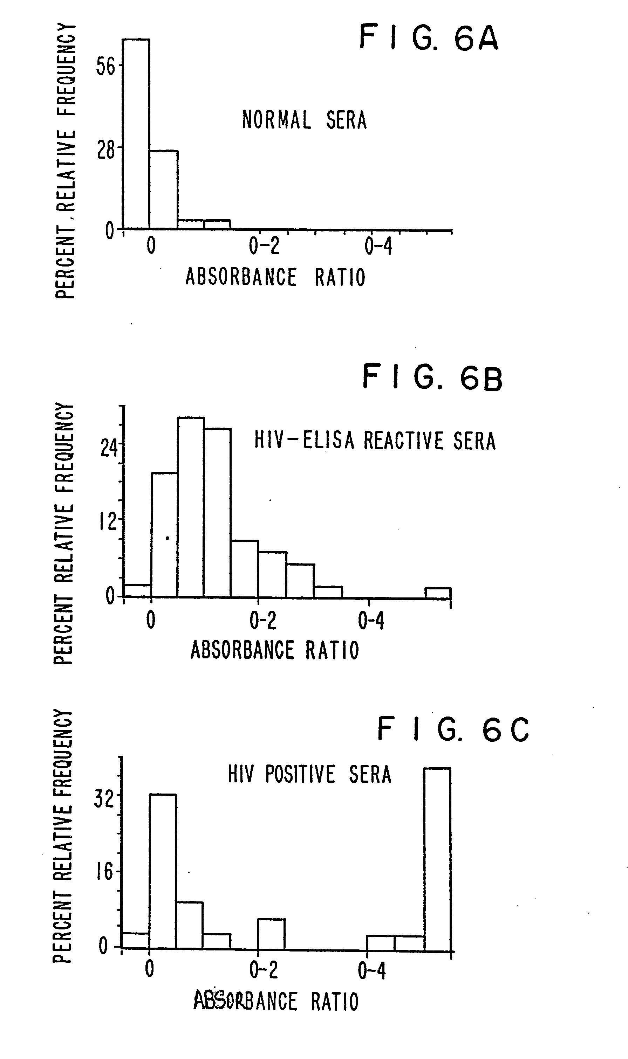

FIGURE 6: Frequency distribution of GAG55-ELISA reactivity in three sample populations. Histograms show the fractions of samples in the absorbance ranges indicated on the horizontal axis. Absorbance ratio is ratio of the sample absorbance to that of a reference HIV positive control serum. Samples with absorbance ratios greater than 0.5 are grouped together for clarity. Upper panel: 69 normal HIV-ELISA-nonreactive blood donors. Middle panel: 57 representative HIV-ELISA reactive env-nonreactive sera from a blood donor population. Lower panel: 31 HIV positive sera: 26/31 of these sera are from patients with clinically diagnosed AIDS or AIDS related complex. Statistics for the populations were: normal sera, mean absorbance ratio = 0.00, s.d. = 0.03; HIV-reactive blood donors, mean = 0.13, s.d. = 0.11; HIV positives, mean = 0.37, s.d. = 0.43.

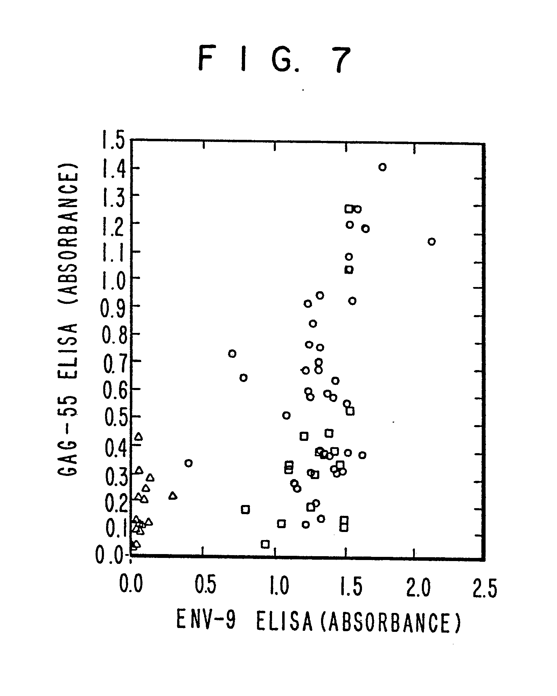

FIGURE 7: Comparison of apparent HIV gag-specific and env-specific antibody levels in human sera analyzed by ELISA. Antigens ENV9 and GAG55 were purified following expression in E. coli Symbols: open triangles, HIV-seronegative; open circles, non-AIDS, HIV-seropositive; open squares, AIDS patients HIV-seropositive. Apparent HIV gag-specific antibody levels are significantly reduced in AIDS patient sera compared to non-AIDS HIV-seropositive sera. This apparent relative decrease in gag-specific antibody levels is selective, since env-specific antibody levels are not similarily affected.

## EXAMPLES

The invention is further described by the following Examples, wherein all parts and percentages are by weight and degrees are Celsius.

## EXAMPLE 1

Plasmid Constructions

HIV sequences were obtained from the proviral clone λ-BH10 (Ratner et al., op. cit.). The HIV-containing SacI fragment of clone λ-BH10 was isolated and treated with DNAaseI under conditions of limited digestion to generate random DNA fragments. The randomly cut HIV DNA fragments were blunt ended by Klenow reaction, ligated to a HindIII linker, cut with HindIII, and inserted into the unique HindIII site of plasmid pTORF2. Plasmid pTORF2 is derived from pBR322 (ATCC No. 37017); sequences inserted at the unique HindIII site are transcribed under the control of an inducible E. coli tryptophan operator and promoter (Ivanoff et al., op. cit.). The resulting plasmid constructions were used to transform E. coli and transformants containing HIV-derived sequences were identified by colony hybridization using nick translated HIV$_{BH10}$ proviral DNA as a probe. Three such plasmids containing HIV-derived sequences are designated pGAG3, pGAG4, PR2. pGAG3, pGAG4, and PR2 were shown by DNA sequence analysis to contain HIV-derived DNA inserts corresponding to nucleotides 227 to 1010, 273 to 1063, and 629 to 2037, respectively. HIV nucleotides are numbered according to Ratner et al. (op. cit.). The HIV$_{BH10}$ gag coding sequence is contained within nucleotides 334 to 1869 (Figure 1). Thus, pGAG3 and pGAG4 contain the 5' end of the gag coding sequence and the HIV-derived insert in pR2 extends beyond the 3' end of the gag coding sequence.

Plasmids pGAG9 and pGAG10, which specify the expression in E. coli under tryptophan promoter control of the complete HIV$_{BH10}$ gag coding sequence, were derived as described below. pGAG9 was derived by ligation of the following DNA fragments: 1) the PstI to PstI tryptophan promoter-containing fragment of pGAG4, 2) the PstI to PstI pBR322 origin-containing fragment from pR2. pGAG10 was similarly derived by ligation of the following DNA fragments: 1) the Pst to PstI tryptophan promoter-containing fragment of pGAG3, 2) the PstI to PstI pBR322 origin-containing fragment of pR2. In this way the PstI site within the gag coding sequence was used to combine the 5' of the gag-coding sequence from pGAG3 and pGAG4 with the 3' of the gag coding sequence of pR2, to generate pGAG10 and pGAG9, respectively. pGAG10 places HIV sequence from nucleotides 227 to 2037 under transcriptional control of the tryptophan promoter. pGAG9 places HIV sequence from nucleotides 273 to 2037 under tryptophan promoter control (see Figure 1). E. coli translational control signals for translation initiation at the gag ATG at nucleotide 334 are provided by the HIV sequences immediately 5' of the gag ATG. These translation initiation signals were predicted to be functional in E. coli on inspection of the HIV sequences.

Plasmid pGAG55 (Figure 2) is constructed by ligation of the following three DNA segments: 1) the large P$_L$-containing BamHI to NcoI fragment from pBF106; 2) the HIV gag-containing ClaI to BamHI DNA segment from pGAG9 or pGAG10; 3) a NcoI to ClaI oligonucleotide of the following sequence:
5'- C ATG GGT GCT AGA GCG TCA GTA TTA AGC GGG GGA GAA TTA GAT-3' 3'- CCA CGC TCT CGC AGT CAT AAT TCG CCC CCT CTT AAT CTA GC-5' The NcoI to ClaI oligonucleotide contains sequence information coding for the first 14 N-terminal amino acids of gag. Plasmid pGAG55 thereby contains the complete HIV$_{BH10}$ gag coding sequence fused to the synthetic E. coli translation initiation signal in plasmid pBF106, as described previously (see p. 14). Plasmid pGAG55 is also referred to as pBF128. A unique NcoI site has been engineered at the translation start ATG of gag without altering the gag coding sequence (see Figure 2). The complete gag coding sequence can now be conveniently moved from GAG55 as a NcoI to BamHI DNA fragment cassette to other expression vectors. For example, the gag coding sequence can be precisely fused to signals directing the expression of the full length gag precursor in mammalian cells. Importantly, HIV sequences 5' to the gag coding sequence have been eliminated in the NcoI to BamHI DNA cassette. HIV sequences immediately 5' to the gag coding sequence, including a splice donor signal, are very likely to limit expression of gag in mammalian cells.

Plasmid GAG17, which specifies the expression in E. coli of the mature, non-fused p17^gag protein, was constructed by ligation of the following three DNA sequences: 1) the pBR322-derived, approximately 2962 base pair SalI to PstI DNA fragment from pGAG55 (see Figure 2); 2) a PstI to PvuII fragment from pGAG55 (approximately 3068 base pair) containing pBR322-derived DNA, lambda-derived DNA, and the 5′ region of HIV gag (see Figure 2); 3) a chemically synthesized PvuII to SalI DNA segment of the following sequence (only 1 DNA strand is shown):

5′- CT GAC ACA GGA CAC AGC AGT CAG GTC AGC CAA AAT TAC TAA G -3′ This PvuII to SalI fragment extends the gag coding sequence from the PvuII site at nucleotide 692 (Figure 1) and introduces of TAA stop codon after the Tyr codon (TAC) at the p17^gag-p24^gag junction (Figure 1). The 3′ G residue of the indicated sequence is the first nucleotide of a SalI site. In pGAG17, a coding sequence corresponding precisely to nucleotides 334 to 729 of HIV$_{BH10}$ (Figure 1) is placed under transcriptional and translational signals derived from pBF106. pBF106 is described previously.

EXAMPLE 2

Reactivity of E. coli-expressed GAG55 with HIV-Specific Antibodies

As shown in Figure 3, GAG55 protein can be efficiently expressed at high levels in E. coli, using the appropriate plasmid expression vector system. Analysis of total E. coli proteins by SDS-PAGE and Coomassie blue staining indicates that GAG55 accumulates to a level of approximately 5% of total E. coli protein. This high level of expression of GAG55 facilitates purification of this protein. GAG17 similarly accumulates to high level in E. coli.

Monoclonal antibodies specific to HIV-expressed p17^gag (BT2, Biotech Research Labs) and p24^gag (BT3, Biotech Research Labs) were found to react specifically with GAG55 proteins when tested in immunoblot and ELISA according to methods described previously. In addition, a rabbit antiserum specific for highly purified HIV p24^gag - (obtained from D. Reed, Du Pont Experimental Station) was immunoreactive with E. coli-expressed GAG55. Furthermore, E. coli-expressed GAG55 was shown to be immunoreactive with many human AIDS patient sera using an immunoblot assay (for example, see Figure 4). Similarly, E. coli-expressed GAG17 is immune reactive with p17^gag-

specific monoclonal antibody BT2 and many human AIDS patient sera. These results confirm that viral antigenic sites reside within E. coli-expressed GAG55 and GAG17.

EXAMPLE 3

Use of E. coli-expressed GAG55 as an Immunogen

Another advantage of the antigenic structure of E. coli-expressed GAG55 is the ability of the peptide to illicit HIV-specific antibodies in animals. Purified GAG55 was injected into rabbits and goats and the resulting antisera were assayed by immunoblot and ELISA, according to the methods described previously. Goat and rabbit antisera to E. coli-expressed GAG55 reacted with the expected HIV-expressed gag-derived proteins p55^gag, p24^gag, and p17^gag on viral immunoblot. Antisera to GAG55 also reacted with an HIV-ELISA (Du Pont).

The titer of the antisera to E. coli-expressed GAG55 was found to be equal to or greater than antisera to gag proteins purified from HIV in specifically binding to the HIV-expressed gag proteins. This result demonstrates that E. coli-expressed GAG55 contains the major native viral epitopes and that antibodies to GAG55 are sensitive reagents for detecting viral proteins.

EXAMPLE 4

Detection of Antibodies to HIV gag in Human Sera

The E. coli-expressed gag-derived protein GAG55 is comprised of protein sequences covering the complete gag open reading frame and should, therefore, be very similar antigenicaly to P55^gag expressed by HIV in infected mammalian cells. In order to compare the ability of GAG55 and HIV-expressed gag antigens to be recognized by human antibodies in HIV-seropositive sera, experiments involving competition between recombinant and viral gag antigens for reaction with serum antibodies were carried out. Using a competition immunoblot technique, six HIV-seropositive human sera were tested for reactivity with GAG55 antigen as described below. Sera were pre-absorbed with GAG55-containing E. coli extract and, in parallel, with a control E. coli extract. These absorptions were followed by analysis of the sera using HIV antigen strips (Biotech Research Labs). With each HIV positive serum tested, recombinant gag polyprotein was found to substantially, if not completely, out-compete reactivity at all the gag-related immunoblot bands (see Figure 5). The specificity of this competition effect for gag was shown by the lack of competition of env or pol bands and by the lack of effect of control E. coli extracts on any HIV immunoblot band. The present results demonstrate that most antibodies in HIV-seropositive sera reacting with HIV gag immunoblot bands also react with GAG55 antigen. Thus, E. coli-expressed GAG55 is clearly a useful reagent for the detection of HIV gag-specific antibodies and, therefore, should be a useful component of HIV diagnostic and blood screening procedures. These procedures may involve, for example, immunoblot or ELISA methods.

## EXAMPLE 5

### Purification of gag-specific antibodies in human HIV-seropositive serum by affinity chromatography using E. coli-expressed GAG55

E. coli-expressed GAG55 was demonstrated using immunoblot assays to bind to essentially all of the gag-specific antibodies in many human HIV-seropositive sera (Example 4, Figure 5). Therefore, we prepared an affinity column for the preparative isolation of HIV gag-specific antibodies. GAG55 was partially purified as described above and covalently coupled to a Reacti-gel support (Pierce Chemical, Rockford, IL) as specified by the supplier. Human serum was loaded to a column containing the GAG55 affinity resin, the column was washed extensively using PBS, the gag-retained human antibodies were eluted at low pH, and the eluted fractions were pH neutralized. Immunoblot analysis of HIV antigens (Biotech Research Labs, Rockville, MD) using the GAG55-purified human antibodies revealed selective immunoreactivity of these antibodies with gag-derived antigens. These results demonstrate the utility of E. coli-expressed gag protein for the preparative isolation of gag-specific antibodies.

HIV gag-specific antibodies will be useful research reagents for the identification and mapping of gag-specific epitopes and their role in the host immune response to HIV. For example, it may be possible to identify gag-specific antibodies that can neutralize or block HIV infection. The availability of such HIV- neutralizing antibodies as therapeutic reagents will depend on efficient procedures for their purification. As discussed previously a full length gag protein, such as E. coli-expressed GAG55, that represents essentially all of the gag-specific epitopes may be the preferred affinity reagent for the identification and purification of gag-specific antibodies. Subfragments of gag protein, as previously disclosed by others, can only represent a subset of the total set of gag-specific epitopes.

## EXAMPLE 6

### Identification of HIV gag-reactive, env- and pol-nonreactive Sera

ELISAs utilizing as antigen purified HIV grown in tissue culture (HIV-ELISA) have proved valuable for screening blood for evidence of exposure to HIV. Many times sera will repeatedly test positive on HIV-ELISA but will not show clear evidence of HIV exposure when subsequently examined by HIV-immunoblot. During screening of blood donors, we and others [Courouce et al., Lancet II:921-922 (1986); Biberfeld et al., Lancet II:289-298 (1986)] often detect reactivity in HIV immunoblots at positions of gag antigens p15, p17, p24, or p55 unaccompanied by detectable immunoreactivity with env. Examples of such HIV gag-reactive, env-nonreactive sera are shown in Figure 5. The specificity of these human antibodies for HIV gag was demonstrated by competition binding with GAG55. Thus, preincubation of the sera with GAG55 specifically blocked binding of the antibody to the gag proteins on the HIV-immunoblot.

More evidence demonstrating the occurence of anti-gag antibodies and quantitative data on their relative levels in different populations was obtained using purified recombinant antigens in ELISA format as shown in Figure 6. The HIV-ELISA-reactive blood donors are distinct from the normal group in having elevated gag ELISA titres, as does the HIV-positive group. In addition, the GAG55-ELISA values for the HIV-ELISA-reactive blood donors are in the same range as several of the HIV-positive sera. Analysis of these samples with the ENV9-ELISA revealed that the absorbances of the HIV-ELISA reactive donor group were distinctly lower than the HIV-positive group and similar to those of normal sera.

For a high percentage of the more than 200 HIV-ELISA positive sera that we have examined, there is unequivocal evidence of antibodies that are immunoreactive with HIV gag antigens, unaccompanied by evidence of HIV env reactivity. The methods demonstrated utilizing both GAG55-ELISA and ENV9-ELISA should prove useful in routine blood screening and diagnostic analysis.

The ability to identify such HIV gag-seropositive and env-seronegative human serum samples is of considerable importance. For example, several studies, including that of A. Saah et al., presented at the National Institutes of Health Consensus Development Conference on "Impact of Routine HTLV-III Antibody Testing on Public Health" (July 7-9, 1986), have indicated that HIV infected individuals who initially display immunoblot reactivity only to gag specific bands may subsequently display definite HIV seropositivity as evidenced by env reactivity. It is also possible that such HIV gag-reactive, env-nonreactive sera reflect exposure to other human retroviruses, such as HTLV-I, HTLV-II or HIV-II, and possibly unrecognized human retroviruses. The significance to the donor and transfusion recipient of such HIV gag-reactive, env-nonreactive sera remains to be determined and further studies are essential. As a interim measure, blood from donors with these laboratory findings should not be transfused. This example illustrates both the importance of methods for the detection of HIV gag proteins and the utility of GAG55 for this purpose.


EXAMPLE 7


Analysis of Human Antibodies to HIV Using E. coli-Expressed env- and gag-derived Antigens

Although the HIV ELISA is a powerful and sensitive tool for screening of human sera for the presence of HIV-specific antibodies, the assay utilizing total virus antigens does not provide information concerning which component or components of the virus are immunoreactive with a given sera. Immunoblot or immunoprecipitation methods can be used to identify immunoreactivity to specific viral components. To date, the definition of HIV-seropositivity is based on immunoblot reactivity with both gag- and env-derived antigens.

As an alternative to immunoblot and immunoprecipitation methods for the analysis of relative levels of antibodies specific to HIV gag and env proteins, we have developed gag- and env-specific ELISAs, using E. coli-expressed GAG55 and ENV9 as antigens. ENV9 contains amino acids corresponding to the 46 amino acids of the C-terminus of the HIV$_{BH10}$ env gp120 domain and 240 amino acids of the N-terminus of the gp41 domain. Methods for detection and quantitation of the relative levels of antibodies to specific, individual antigenic components of HIV, such as gag- and env-encoded proteins, provides information that may be useful for the diagnosis and prognosis of HIV-infected individuals. The ELISA format provides considerable advantages compared to immunoblot and immunoprecipitation methods. The ELISA procedure is sensitive, quantitative, rapid, non-radioactive, and relatively inexpensive and simple to run.

Recently several ELISA assays have been developed which utilize as antigen proteins expressed in E. coli to detect antibodies to the AIDS virus [Dowbenko, et al., Proc. Natl. Acad. Sci. 82: 7748-7752 (1985); Steimer, et al., Virology 150:283-290 (1986); Cabradilla et al., Biotechnology 4: 128-133 (1986); Chang et al., Biotechnology 3:905-909 (1985); Shoeman et al., Anal. Biochem. 161:370-379 (1987)]. Previous recombinant gag-derived ELISAs which have been described only contain subregions of gag, such as p24$^{gag}$, and do not contain sequences corresponding to the entire gag coding sequence. Since the GAG55 represents the complete gag coding sequence, the GAG55-ELISA may detect HIV gag-specific antibodies in human sera that are not detected by ELISAs employing subregions of gag. In particular, GAG55 is preferred to other reported recombinant gag antigens because the complete p17$^{gag}$ region is represented. In addition, GAG55 and GAG17 do not contain any non-HIV or non-HIV gag amino acid sequences. These antigens are preferred for antibody detection because of the specificity for gag and of the reduced probability of detecting false-positive, non-HIV immunoreactivity.

The HIV gag-specific and env-specific antibody levels were quantitated in human sera from individuals of known clinical status, using both the GAG55-ELISA and ENV9-ELISA. Sera categorized as being from AIDS patients, HIV-seropositive (non-AIDS), and HIV-seronegative individuals were obtained from Duke University. These sera were analyzed using the GAG55 and ENV9 ELISAs. The results of these assays are shown in Figure 7.

As shown in Figure 7, the gag-specific apparent antibody levels in many of the AIDS patient sera are reduced compared to the gag-specific apparent antibody levels in non-AIDS, HIV-seropositive sera. This effect is selective, since the HIV env-specific antibody levels do not distinguish the AIDS and non-AIDS seropositive groups. The GAG55-ELISA alone is obviously limited as an HIV screening assay since many of the characterized HIV positive sera were not detected as positive (see also Example 6, Figure 6). This does not, however, preclude the use of a gag-specific ELISA as an important component of diagnostic assays. For example, many sera exhibit HIV gag immunoreactivity without detectable env-specific immunoreactivity (Example 6). Moreover, quantitation of the relative antibody levels specific to gag and env and other HIV antigenic components may be useful for monitoring HIV-associated disease. The results presented indicate that it may be possible to develop a correlation between the serum reactivity on the GAG55 and ENV9 ELISA assays and the stage of disease. The present data provide evidence that quantitation by ELISA of the relative HIV env-specific and gag-specific antibody levels in human sera is useful for both screening and diagnostic analysis of human sera.

**Claims**

1. A recombinant plasmid, capable of high level expression in E. coli of a nonfused peptide corresponding to the complete HIV gag coding sequence and exhibiting the antigenicity of the complete HIV p55$^{gag}$ protein, consisting essentially of plasmid pBR322 and DNA coding for amino acids 1 to 512 of HIV protein p55$^{gag}$, with a translation start signal and an ATG/Met translation start codon downstream from a promoter selected from the group consisting of the E. coli lactose operon promoter, the E. coli tryptophan operon promoter, and the phage lambda $P_L$ promoter.

2. A recombinant plasmid of Claim 1 having incorporated therein a translation start signal which is an HIV sequence immediately 5′ of the gag ATG/Met translation start codon or an oligonucleotide linker as defined in Example 1.

3. A recombinant plasmid of Claim 1 wherein the gag ATG/Met translation start codon is part of an NcoI restriction endonuclease site.

4. An E. coli cell transformed with a plasmid of anyone of claims 1 to 3.

5. An HIV gag-encoded peptide expressed by a cell of Claim 4.

6. A recombinant plasmid, capable of high level expression in E. coli of a nonfused peptide corresponding to the complete mature HIV p17$^{gag}$ coding sequence and exhibiting the antigenicity of the complete HIV p17$^{gag}$ peptide, consisting essentially of plasmid pBR322 and DNA coding for amino acids 1 to 132 of HIV peptide p55$^{gag}$, with a translation start signal and an ATG/Met translation start codon downstream from a promoter selected from the group consisting of the E. coli lactose operon promoter, the E. coli tryptophan operon promoter, and the phage lambda $P_L$ promoter.

7. A recombinant plasmid of Claim 6 having incorporated therein a translation start signal which is an HIV sequence immediately 5′ of the gag ATG/Met translation start codon or an oligonucleotide linker as defined in Example 1.

8. A recombinant plasmid of Claim 6 wherein the gag ATG/Met translation start codon is part of an NcoI restriction endonuclease site.

9. An E. coli cell transformed with a plasmid of anyone of claims 6 to 8.

10. An HIV gag-encoded peptide expressed by a cell of Claim 9.

11. An E. coli-expressed recombinant nonfused peptide corresponding to the complete HIV p55$^{gag}$ coding sequence and exhibiting the antigenicity of the complete HIV p55 $^{gag}$ protein.

12. An E. coli-expressed recombinant nonfused peptide according to Claim 11 which is encoded by nucleotides numbered 334 to 1869 of the HIV genome, as shown in Figure 1.

13. An E. coli-expressed recombinant nonfused peptide corresponding to the complete p17 subregion of the HIV p55$^{gag}$ coding sequence, and exhibiting the antigenicity of the complete HIV p17$^{gag}$ protein.

14. An E. coli-expressed recombinant nonfused peptide according to Claim 13 which is encoded by nucleotides numbered 334 to 729 of the HIV genome, as shown in Figure 1.

15. In a diagnostic kit used for detecting antibodies to HIV in a biological sample wherein the sample is contacted with a peptide and immunoreactivity is detected, the improvement comprising employing a peptide of anyone of claims 5, 10, 11, and 13.

16. In a process for detecting antibodies to HIV in a biological sample, comprising contacting said sample with a peptide which is immunoreactive with said antibodies and detecting immunoreactivity, the improvement comprising employing a peptide of anyone of claims 5, 10, 11, and 13.

17. A vaccine protective against HIV comprising an effective protective amount of a peptide of anyone of claims 5, 10, 11, and 13 in a pharmaceutically acceptable carrier.

# F I G. IA

```
                                        228                                      255
GAA AGC GGA AAG GGA AAC CAG AGC TCT CTC GAC GCA GGA CTC GGC TTG CTG AAG
Glu Ser Gly Lys Gly Asn Gln Ser Ser Leu Asp Ala Gly Leu Gly Leu Leu Lys


                                        282                                      309
CGC GCA CGG CAA GAG GCG AGG GGC GGC GAC TGG TGA GTA CGC CAA AAA TTT TGA
Arg Ala Arg Gln Glu Ala Arg Gly Gly Asp Trp  .  Val Arg Gln Lys Phe  .


                                        336                                      363
CTA GCG GAG GCT AGA AGG AGA GAG ATG GGT GCG AGA GCG TCA GTA TTA AGC GGG
Leu Ala Glu Ala Arg Arg Arg Glu MET Gly Ala Arg Ala Ser Val Leu Ser Gly


                                        390                                      417
GGA GAA TTA GAT CGA TGG GAA AAA ATT CGG TTA AGG CCA GGG GGA AAG AAA AAA
Gly Glu Leu Asp Arg Trp Glu Lys Ile Arg Leu Arg Pro Gly Gly Lys Lys Lys


                                        444                                      471
TAT AAA TTA AAA CAT ATA GTA TGG GCA AGC AGG GAG CTA GAA CGA TTC GCA GTT
Tyr Lys Leu Lys His Ile Val Trp Ala Ser Arg Glu Leu Glu Arg Phe Ala Val


                                        498                                      525
AAT CCT GGC CTG TTA GAA ACA TCA GAA GGC TGT AGA CAA ATA CTG GGA CAG CTA
Asn Pro Gly Leu Leu Glu Thr Ser Glu Gly Cys Arg Gln Ile Leu Gly Gln Leu


                                        552                                      579
CAA CCA TCC CTT CAG ACA GGA TCA GAA GAA CTT AGA TCA TTA TAT AAT ACA GTA
Gln Pro Ser Leu Gln Thr Gly Ser Glu Glu Leu Arg Ser Leu Tyr Asn Thr Val


                                        606                                      633
GCA ACC CTC TAT TGT GTG CAT CAA AGG ATA GAG ATA AAA GAC ACC AAG GAA GCT
Ala Thr Leu Tyr Cys Val His Gln Arg Ile Glu Ile Lys Asp Thr Lys Glu Ala


                                        660                                      687
TTA GAC AAG ATA GAG GAA GAG CAA AAC AAA AGT AAG AAA AAA GCA CAG CAA GCA
Leu Asp Lys Ile Glu Glu Glu Gln Asn Lys Ser Lys Lys Lys Ala Gln Gln Ala


                                        714                                      741
GCA GCT GAC ACA GGA CAC AGC AGT CAG GTC AGC CAA AAT TAC CCT ATA GTG CAG
Ala Ala Asp Thr Gly His Ser Ser Gln Val Ser Gln Asn Tyr Pro Ile Val Gln


                                        768                                      795
AAC ATC CAG GGG CAA ATG GTA CAT CAG GCC ATA TCA CCT AGA ACT TTA AAT GCA
Asn Ile Gln Gly Gln MET Val His Gln Ala Ile Ser Pro Arg Thr Leu Asn Ala


                                        822                                      849
TGG GTA AAA GTA GTA GAA GAG AAG GCT TTC AGC CCA GAA GTA ATA CCC ATG TTT
Trp Val Lys Val Val Glu Glu Lys Ala Phe Ser Pro Glu Val Ile Pro MET Phe
```

# F I G. IB

876           903
```
·TCA GCA TTA TCA GAA GGA GCC ACC CCA CAA GAT TTA AAC ACC ATG CTA AAC ACA
 Ser Ala Leu Ser Glu Gly Ala Thr Pro Gln Asp Leu Asn Thr MET Leu Asn Thr
```

930           957
```
GTG GGG GGA CAT CAA GCA GCC ATG CAA ATG TTA AAA GAG ACC ATC AAT GAG GAA
Val Gly Gly His Gln Ala Ala MET Gln MET Leu Lys Glu Thr Ile Asn Glu Glu
```

984           1011
```
GCT GCA GAA TGG GAT AGA GTA CAT CCA GTG CAT GCA GGG CCT ATT GCA CCA GGC
Ala Ala Glu Trp Asp Arg Val His Pro Val His Ala Gly Pro Ile Ala Pro Gly
```

1038           1065
```
CAG ATG AGA GAA CCA AGG GGA AGT GAC ATA GCA GGA ACT ACT AGT ACC CTT CAG
Gln MET Arg Glu Pro Arg Gly Ser Asp Ile Ala Gly Thr Thr Ser Thr Leu Gln
```

1092           1119
```
GAA CAA ATA GGA TGG ATG ACA AAT AAT CCA CCT ATC CCA GTA GGA GAA ATT TAT
Glu Gln Ile Gly Trp MET Thr Asn Asn Pro Pro Ile Pro Val Gly Glu Ile Tyr
```

1146           1173
```
AAA AGA TGG ATA ATC CTG GGA TTA AAT AAA ATA GTA AGA ATG TAT AGC CCT ACC
Lys Arg Trp Ile Ile Leu Gly Leu Asn Lys Ile Val Arg MET Tyr Ser Pro Thr
```

1200           1227
```
AGC ATT CTG GAC ATA AGA CAA GGA CCA AAA GAA CCT TTT AGA GAC TAT GTA GAC
Ser Ile Leu Asp Ile Arg Gln Gly Pro Lys Glu Pro Phe Arg Asp Tyr Val Asp
```

1254           1281
```
CGG TTC TAT AAA ACT CTA AGA GCC GAG CAA GCT TCA CAG GAG GTA AAA AAT TGG
Arg Phe Tyr Lys Thr Leu Arg Ala Glu Gln Ala Ser Gln Glu Val Lys Asn Trp
```

1308           1335
```
ATG ACA GAA ACC TTG TTG GTC CAA AAT GCG AAC CCA GAT TGT AAG ACT ATT TTA
MET Thr Glu Thr Leu Leu Val Gln Asn Ala Asn Pro Asp Cys Lys Thr Ile Leu
```

1362           1389
```
AAA GCA TTG GGA CCA GCG GCT ACA CTA GAA GAA ATG ATG ACA GCA TGT CAG GGA
Lys Ala Leu Gly Pro Ala Ala Thr Leu Glu Glu MET MET Thr Ala Cys Gln Gly
```

1416           1443
```
GTA GGA GGA CCC GGC CAT AAG GCA AGA GTT TTG GCT GAA GCA ATG AGC CAA GTA
Val Gly Gly Pro Gly His Lys Ala Arg Val Leu Ala Glu Ala MET Ser Gln Val
```

**24**

1470           1497
```
ACA AAT ACA GCT ACC ATA ATG ATG CAG AGA GGC AAT TTT AGG AAC CAA AGA AAG
Thr Asn Thr Ala Thr Ile MET MET Gln Arg Gly Asn Phe Arg Asn Gln Arg Lys
```

1524           1551
```
ATG GTT AAG TGT TTC AAT TGT GGC AAA GAA GGG CAC ACA GCC AGA AAT TGC AGG
MET Val Lys Cys Phe Asn Cys Gly Lys Glu Gly His Thr Ala Arg Asn Cys Arg
```

# F I G. IC

1578               1605
GCC CCT AGG AAA AAG GGC TGT TGG AAA TGT GGA AAG GAA GGA CAC CAA ATG AAA
Ala Pro Arg Lys Lys Gly Cys Trp Lys Cys Gly Lys Glu Gly His Gln MET Lys

1632               1659
GAT TGT ACT GAG AGA CAG GCT AAT TTT TTA GGG AAG ATC TGG CCT TCC TAC AAG
Asp Cys Thr Glu Arg Gln Ala Asn Phe Leu Gly Lys Ile Trp Pro Ser Tyr Lys

1686               1713
GGA AGG CCA GGG AAT TTT CTT CAG AGC AGA CCA GAG CCA ACA GCC CCA CCA TTT
Gly Arg Pro Gly Asn Phe Leu Gln Ser Arg Pro Glu Pro Thr Ala Pro Pro Phe

1740               1767
CTT CAG AGC AGA CCA GAG CCA ACA GCC CCA CCA GAA GAG AGC TTC AGG TCT GGG
Leu Gln Ser Arg Pro Glu Pro Thr Ala Pro Pro Glu Glu Ser Phe Arg Ser Gly

1794               1821
GTA GAG ACA ACA ACT CCC CCT CAG AAG CAG GAG CCG ATA GAC AAG GAA CTG TAT
Val Glu Thr Thr Thr Pro Pro Gln Lys Gln Glu Pro Ile Asp Lys Glu Leu Tyr

1848               1875
CCT TTA ACT TCC CTC AGA TCA CTC TTT GGC AAC GAC CCC TCG TCA CAA TAA AGA
Pro Leu Thr Ser Leu Arg Ser Leu Phe Gly Asn Asp Pro Ser Ser Gln . Arg

1902               1929
TAG GGG GGC AAC TAA AGG AAG CTC TAT TAG ATA CAG GAG CAG ATG ATA CAG TAT
. Gly Gly Asn . Arg Lys Leu Tyr . Ile Gln Glu Gln MET Ile Gln Tyr

1956               1983
TAG AAG AAA TGA GTT TGC CAG GAA GAT GGA AAC CAA AAA TGA TAG GGG GAA TTG
. Lys Lys . Val Cys Gln Glu Asp Gly Asn Gln Lys . . Gly Glu Leu

2010
GAG GTT TTA TCA AAG TA
Glu Val Leu Ser Lys

# F I G. 2

EP 0 293 792 A2

# F I G. 3

1    2    3

—92.5

—66.2

←

—45

—31

—21.5

# F I G. 4

1  2  3

—97.4

—68

—43

—25.7

—18.4

# F I G. 5

F I G. 6A

NORMAL SERA

PERCENT, RELATIVE FREQUENCY

56

28

0

0        0-2        0-4

ABSORBANCE RATIO

F I G. 6B

HIV-ELISA REACTIVE SERA

PERCENT RELATIVE FREQUENCY

24

12

0

0        0-2        0-4

ABSORBANCE RATIO

F I G. 6C

HIV POSITIVE SERA

PERCENT RELATIVE FREQUENCY

32

16

0

0        0-2        0-4

ABSORBANCE RATIO

# F I G. 7